# EUROPEAN PATENT SPECIFICATION

(11) **EP 2 710 902 B1**
(45) Date of publication and mention of the grant of the patent: **25.12.2019**
(21) Application number: 12807257.6
(22) Date of filing: 03.07.2012
(51) Int. Cl.: A23L 2/39, A23L 2/52, A23L 2/60, A61K 35/63, A23F 5/14, A23F 5/40, A23F 5/44, A23L 21/20

(54) **COFFEE DRINK IN POWDER FORM AND METHOD FOR PRODUCING IT**
KAFFEEGETRÄNK IN PULVERFORM UND VERFAHREN ZUR HERSTELLUNG
BOISSON À BASE DE CAFÉ SOUS FORME DE POUDRE ET PROCÉDÉ DE PRODUCTION

(30) Priority: 05.07.2011 RU 2011128763
(43) Date of publication of application: 26.03.2014
(73) Proprietor: Obshestvo S Ogranichennoj Otvetstvennostju "PARAFARM", Penza 440026 (RU)
(72) Inventor: TRIFONOV, Vyacheslav Nikolaevich, Zarechni 442960 Penzenskaya obl. (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440061 (RU); HOMYKOVA, Irina Vladimirovna, Penza 440003 (RU); ELISTRATOVA, Tatiana Viktorovna, Penza 440018 (RU)
(74) Representative: Engel, Christoph Klaus
(86) International application number: PCT/RU2012/000527
(87) International publication number: WO 2013/006093

(56) References cited:
- WO-A1-2011/046470
- CN-A- 1 045 276
- CN-A- 1 325 718
- CN-A- 101 152 239
- CN-A- 101 444 244
- JP-A- 2006 197 911
- JP-A- 2010 051 313
- RU-C1- 2 258 522
- RU-C1- 2 312 670
- RU-C1- 2 390 270
- RU-C2- 2 245 155
- RU-C2- 2 279 227
- US-A- 5 882 716

## Description

The invention relates to the food industry and to medicine, in particular to biologically active dietary supplements (BADSs). It is intended for prophylaxis and therapeutic prophylaxis in conditions associated with fatigue.

Currently, no drone brood-based coffee or other drinks with tonic properties are produced in Russia or other countries.

CN 1 325 718 A discloses a complex composition, which includes royal jelly, propolis, worker bee, Epimedium, Cynomorium, Astragalus, Red Ginseng, and other medicinal herbs. Propolis can not be added to a coffee powder because of its strong and specific taste that would not like to consumers of the beverage.

CN 101 444 244 A discloses complex composition of a tee substitute consisting of tee from tee leaves of jasmine, peaches, roses, chrysanthemums, osmanthus, natural honey, pollen, propolis, royal jelly and other ingredients.

JP 2006 197 911 A discloses composition of a complex food additive consisting of bee pollen, bee or drone larvae, beer yeast, algae, fine powder of brown rice sprouts, extract of yamabushi mushroom, cinnamon, titanium dioxide, coenzyme Q-10. The composition is used for recovery from fatigue. This document does not disclose a method of preservation of drone brood. The composition contains ingredients, which can not be dissolved in water, e.g. pollen.

JP 2010 051 313 A describes a composition for oral ingestion containing larva of bee, propolis, and royal jelly. This composition shall have a synergistic antioxidative activity.

A "Biologically Active Supplement for the Manufacture of Cosmetic Products with Regenerative Activity" was patented in Russia (Patent No. 2128501). This biologically active dietary supplement comprises drone-bee larvae recommended for use in the manufacture of cosmetic products with regenerative properties. A method for producing a biologically active product from drone-bee larvae, or worker bees, or queen bees (Patent No. 74872/1980, OSIM), which is a biologically active agent used in manufacture of medicinal and cosmetic products, was patented in Romania. This product contains lactose, which does not dissolve in water and is a suspension. Also known is Patent No. 2245155 "COMPOSITION OF THE CANNED HOMOGENATE OF THE BEES BROOD (DRONE BROOD AND QUEEN LARVAE)". We regard the substance as a prototype.

The invention is defined by the claims.

Larval homogenate is a readily available cheap and promising source for obtaining prophylaxis and therapeutic prophylaxis products and food products. The neutral environment of brood and the presence of nutritional substances obviously provide favorable conditions for the growth of microorganisms and fungi. This leads to rapid spoilage of the raw materials when they are stored improperly. The addition of sorbic and citric acids to the prototype stabilizes drone brood; however, acids have an adverse effect on the biologically active constituents of drone brood, causing their destruction.

It is proposed to preserve drone brood using glucose or fructose by mixing 1 part of drone-brood homogenate with 3 to 9 parts of glucose and to add this mixture to a coffee or other drink in powder form in order to dissolve the mixture in water.

Experiments have proven that this composition is well stabilized in terms of its physicochemical parameters and allows for long-term storage of this product (for more than a year).

The resulting product reduces fatigue and has an invigorating and ergogenic effect.

Below is an example procedure for making the product.

Drone brood larvae are taken and homogenized with a mixer (or any other similar device) until uniform mass forms. 5 parts of glucose are added to the resulting drone-brood homogenate. The product is thoroughly blended again with a mixer until uniform mass forms. The resulting final product is then added into a coffee drink in powder form with a ratio of 1 to 9 and mixed once again until uniform mass forms. The product is to be used in accordance with the instructions for the coffee drink.

A coffee drink in powder form consists of:
1 part drone brood,
3-9 parts glucose and/or fructose, and
1-100 parts coffee drink in powder form.

Despite the fact that the components that form ingredients of the claimed product are known, their combination in a single product is not known in the prior art, namely the resulting formulation makes it possible to achieve the following objectives:
- preserving and stabilizing the properties of drone brood,
- providing water solubility of the resulting product, and
- achieving the claimed technical result, namely enhancing the prophylaxis and therapeutic prophylaxis effect in conditions associated with fatigue.

## Claims

1. A coffee drink in powder form, consisting of:
1 part Drone brood;
3-9 parts glucose and/or fructose; and
1-100 parts coffee drink in powder form.

2. Method for producing the coffee drink in powder form according to claim 1 comprising the following steps:
• 1 part of Drone brood larvae are blended with a mixer until homogeneous;
• 5 parts of glucose are added to the resulting drone brood homogenate and that mixture is thoroughly blended again with a mixer into a uniform mass;
• the resulting mixture is then added to a coffee drink in powder form in a proportion of 1 to 9 and blended into a uniform mass.

## Patentansprüche

1. Kaffeegetränk in Pulverform, bestehend aus:
1 Teil Drohnenbrut;
3-9 Teile Glucose und/oder Fructose; und
1-100 Teile Kaffeegetränk in Pulverform.

2. Verfahren zum Herstellen des Kaffeegetränks in Pulverform nach Anspruch 1, umfassend die folgenden Schritte:
• 1 Teil Drohnenbrutlarven wird mit einem Rührgerät vermischt, bis er homogen ist;
• 5 Teile Glucose werden dem resultierenden Drohnenbruthomogenat zugegeben und diese Mischung wird wiederum mit einem Rührgerät gründlich zu einer gleichförmigen Masse vermischt;
• die resultierende Mischung wird dann einem Kaffeegetränk in Pulverform in einem Anteil von 1 bis 9 zugegeben und zu einer gleichförmigen Masse vermischt.

## Revendications

1. Boisson à base de café sous forme de poudre, constitué de :
1 partie de couvain mâle ;
3 à 9 parties de glucose et/fructose ; et
1 à 100 parties de boisson à base de café sous forme de poudre.

2. Procédé de production de la boisson base de café sous forme de poudre selon la revendication 1, comprenant les étapes suivantes :
• 1 partie de larves de couvain mâle sont liées avec un mélangeur jusqu'à ce qu'elles soient homogènes ;
• 5 parties de glucose sont ajoutées à l'homogénat de couvain mâle résultant et ce mélange est de nouveau soigneusement mélangé avec un mélangeur en une masse uniforme ;
• le mélange résultant est ensuite ajouté à une boisson à base de café sous forme de poudre dans une proportion de 1 à 9 et mélangé en une masse uniforme.
